# EUROPEAN PATENT SPECIFICATION

(11) **EP 3 773 480 B1**
(45) Date of publication and mention of the grant of the patent: **03.07.2024**
(21) Application number: 19722952.9
(22) Date of filing: 04.04.2019
(51) Int. Cl.: A61K 9/00, A61P 35/00, A61L 31/06, A61L 31/14, A61L 31/16, A61K 47/34

(54) **AN IMPLANTABLE DEVICE FOR LOCALIZED DRUG DELIVERY, USES THEREOF AND A MANUFACTURING METHOD THEREOF**
IMPLANTIERBARE VORRICHTUNG ZUR LOKALISIERTEN WIRKSTOFFFREISETZUNG, IHRE VERWENDUNGEN UND HERSTELLUNGSVERFAHREN DAFÜR
DISPOSITIF IMPLANTABLE POUR L'ADMINISTRATION LOCALISÉE DE MÉDICAMENT, SES UTILISATIONS ET SON PROCÉDÉ DE FABRICATION

(30) Priority: 05.04.2018 IT 201800004220
(43) Date of publication of application: 17.02.2021
(73) Proprietor: Fondazione Istituto Italiano di Tecnologia, 16163 Genova (IT)
(72) Inventor: DECUZZI, Paolo, 16146 Genova (IT); DI MASCOLO, Daniele, 16163 Genova (IT)
(74) Representative: Comoglio, Elena
(86) International application number: PCT/IB2019/052748
(87) International publication number: WO 2019/193524

(56) References cited:
- KR-A- 20170 097 838
- US-A1- 2005 008 675
- US-A1- 2005 084 514
- US-A1- 2008 020 000
- US-A1- 2008 132 922

## Description

The present invention generally refers to an implantable device for localized drug delivery. More specifically, the invention refers to an implantable device for localized drug delivery that has been developed primarily for the treatment of tumors, even more specifically, brain tumors.

Malignant gliomas account for almost 50% of all brain tumors in adults, with over 70,000 new cases diagnosed each year. The current standard of care for this kind of aggressive brain tumor involves surgery to remove the main malignant mass from the brain, followed by chemotherapy and radiation therapy. Despite this intense therapeutic intervention, the overall survival of patients diagnosed with glioma is generally less than 20 months. Such an unfavorable prognosis is associated with the highly infiltrating nature of glioma cells, which cannot be completely removed after surgery, eventually leading to recurrences. Furthermore, a sufficient release of therapeutic agents to the brain is limited by the presence and complexity of the blood-brain barrier (BBB).

For these reasons, polymeric implants have been proposed for localized chemotherapy, which are implanted directly into the surgical site and release their therapeutic content locally. The object of this approach is the pharmacological removal of the residual tumor cells. Among these implants, the Gliadel^{®} wafer is the most effective and widely used. Gliadel^{®} is a cylindrical polymer wafer loaded with carmustine, a drug that interferes with DNA synthesis and stops cell proliferation. This device is described in U.S. Patent 8,895,597, which concerns a device for the intracranial release of chemotherapy for gliomas, or other types of tumors, more preferably, after surgical resection. The matrix of the implantable device is made of natural or synthetic biodegradable polymers. For this purpose, the preferred polymers are polyanhydrides and polyhydroxy acids, in particular poly(lactic acid-glycolic acid) copolymers. The matrix may be prepared in the form of microparticles (e.g. microbeads, microcapsules or nanoparticles), discs and wafers. In particular, wafers are the commercially available solution. Gliadel^{®} is deposited directly in the resection cavity, where it slowly releases the chemotherapeutic active ingredient. However, this device only slightly improves the survival rate of patients, mainly due to the short release time (about 2 weeks) and the shallow penetration of therapeutic molecules in the brain tissue. Moreover, its effectiveness is still controversial among doctors. The studies carried out have failed to clearly demonstrate whether there is a benefit to the overall survival of patients. Moreover, based on meta-analysis studies, Gliadel^{®} appears to be beneficial in patients with newly diagnosed glioma, but not in patients that have demonstrated recurrent glioma. Some clinical studies have also shown toxic side effects that, together with limited survival improvements, exclude the use of Gliadel^{®} in new clinical studies. US 2005/084514 discloses an absorbable mesh surgical material with mesh strands and open spaces, to be placed post-operatively into or around biological tissue at the site of a surgical procedure to form a cytostatic antiproliferative surgical wrap. The mesh strands can be made from biodegradable materials such as poly-lactide or poly-glycolide polymers or copolymers wherein the cytostatic antiproliferative drug is attached.

One purpose of the present invention is thus to provide a localized drug delivery system that has been proven to be effective and that satisfies the shortcomings of the prior art outlined above.

These and other objects are achieved through the implantable device for the localized delivery of one or more pharmaceutical active ingredients having the features defined in the characterizing part of claim 1.

The dependent claims define further beneficial features of the implantable device of the invention and form an integral part of the present description.

The following is a detailed description of the implantable device according to the invention, as well as of the procedure for the manufacture thereof, which is a second aspect of the invention. A third aspect of the invention concerns the therapeutic applications of the implantable device according to the invention.

The implantable device according to the invention, which will hereinafter be referred to for brevity as "µMESH device", allows the localized delivery of a wide variety of therapeutic agents, possibly incorporated in nanoparticles. The particular chemical characteristics and the micrometric geometry of the µMESH device also allow a better integration of the same device with the surrounding tissue after implantation. Although the µMESH device may be used for the localized treatment of various diseases, such as cancer, diabetes and cardiovascular diseases, it is particularly beneficial in the treatment of brain tumors, such as glioblastoma multiforme (GMB). In this field, the implantation of the µMESH device in the surgically resected tumor area may in effect lead to stopping the recurrence of the tumor.

Further features and advantages of the invention will become apparent from the detailed description that follows, provided by way of non-limiting example.

In this description, the following terms are used with the meaning specified below.

"Biodegradable", with reference to a material, means that said material is able to be metabolized naturally and in a non-harmful way.

"Biocompatible", with reference to a material, means that said material has no toxic or harmful effects on biological functions.

"Net" or "grid" interchangeably refers to the general shape of a polymer matrix of micrometric "meshes" or "cells" defining respective through holes.

The phrase "thickness of the polymer matrix" is used to indicate the size of the polymer matrix along a direction parallel to the axis of the holes.

The expression "size of the through holes" means: in the case of circular holes, the diameter of the hole itself; in the case of holes in the shape of regular polygons, the diameter of the inscribed circumference; and in the case of rectangular holes, the minor axis of the inscribed ellipse.

This dimension is measured in a direction perpendicular to the axis of the hole and in the plane of the hole itself.

As mentioned above, the present invention concerns an implantable device for the localized delivery of one or more active pharmaceutical ingredients, the device comprising a polymer matrix having a general net or grid shape, comprising a plurality of micrometric meshes, or micrometric cells, having a predefined geometric shape. All the micrometric meshes of the grid have the same geometric shape and define respective through holes.

The polymer matrix of the µMESH device consists of one or more biodegradable and biocompatible polymers, which may be natural or synthetic. The polymer matrix also includes one or more pharmaceutical active ingredients.

Polymers suitable for producing the biodegradable and biocompatible polymer matrix of the implantable device of the invention are, by way of non-limiting example, poly(lactic-co-glycolic acid) (PLGA), polyethylene glycol (PEG), hyaluronic acid (HA), chitosan, polymethyl methacrylate (PMMA), polylactic acid (PLA), polyglycolic acid (PGA), polycaprolactone (PCL) and combinations thereof. Preferably, the polymer matrix consists of or comprises PLGA.

In a preferred embodiment, the polymer matrix is supported on a polymeric support layer consisting of one or more biodegradable and biocompatible polymers. By way of non-limiting example, polymers suitable for making the support layer are polyvinyl alcohol (PVA), gelatin, chitosan, N-(2-hydroxypropyl) methacrylamide (HPMA), hydroxypropyl methylcellulose (HPMC), poly(lactic-co-glycolic acid) (PLGA), polyethylene glycol (PEG), hyaluronic acid (HA), chitosan, polymethyl methacrylate (PMMA), polylactic acid (PLA), polyglycolic acid (PGA), polycaprolactone (PCL) and combinations thereof. Preferably, the support layer consists of or comprises polyvinyl alcohol (PVA).

The active pharmaceutical ingredients suitable for use in the µMESH device of the invention are any substance that may provide a therapeutic effect on a disease, such as anti-tumor agents (taxane, agent of the anthracycline family), protease inhibitors, reverse transcriptase inhibitors, biomolecules (RNA, antibodies, peptides), iron oxide nanoparticles, gold nanoparticles, quantum dots, solid lipid nanoparticles, polymer nanoparticles and nanoparticles loaded with drugs or biomolecules. The µMESH device of the invention may also encapsulate anti-bacterial, anti-viral and anti-fungal agents, a vaccine, an agent active against anti-autoimmune diseases, a psychotherapeutic agent, a cardiovascular active ingredient, a blood modifier, a gastrointestinal active ingredient, an active agent against respiratory diseases, an anti-arthritic agent, an active agent against diabetes, an anticonvulsant, a bone metabolism regulator, an active agent against multiple sclerosis, a hormone, an active agent on the urinary tract, an immunosuppressant, an ophthalmic product, a sedative, an active ingredient for the treatment of a sexual dysfunction, an anesthetic, an active agent against migraine, an active ingredient against infertility, a weight control product and any combination thereof.

In a preferred embodiment, the pharmaceutical active ingredient is a chemotherapy such as Docetaxel (DTXL), Paclitaxel, Taxotere, Vorinostat, Irinotecan, Topotecan, Methotrexate, Carboplatin, Cisplatin, Oxaliplatin, Vinblastine, Vincristine, Carmustine, Temozolomide (TMZ), Doxorubicin, Daunorubicin or any combination thereof.

The selected active ingredient may be appropriately incorporated into the polymer matrix in a form suitable for obtaining a prolonged release of the main active ingredient itself, without affecting the pharmacological activity thereof. For example, the Docetaxel incorporated in the polymer matrix in the form of powder is released up to 60 days after implantation. Longer release times may be easily achieved by incorporating the active ingredient, instead of in the form of powder, in its co-crystalline form. This solid form reduces the rate of dissolution, which is a necessary prerequisite for subsequent diffusion into the space surrounding the implanted device, reducing the general release profile.

The chemotherapy active ingredient may also be incorporated into the polymer matrix in combination with another active ingredient with a different therapeutic purpose, such as a biomolecule, an anti-inflammatory active ingredient, a cytokine, an anti-proliferative active ingredient, a chemokine to modulate the migratory processes of immune cells, an active ingredient stimulating the immune system, an anti-fungal agent or an anti-bacterial agent. Alternatively, the additional active ingredient may be incorporated into the polymeric support layer. Particularly preferred examples of active ingredients to be used in combination with a chemotherapy are diclofenac, celecoxib, curcumin, ibuprofen, indomethacin, acetylsalicylic acid, zoledronic acid.

In a further embodiment, the active ingredient incorporated in the polymer matrix and/or polymer support layer is conjugated with or included in a nanoparticle. By way of non-limiting example, iron oxide nanoparticles, gold nanoparticles, quantum dots, micelles, liposomes, solid lipid nanoparticles, polymeric nanoparticles are cited. These nanoparticles may be loaded with any one of the aforementioned active ingredients and the combinations thereof.

As mentioned above, the polymer matrix of the µMESH device has a general net or grid shape and comprises a plurality of micrometric meshes, or micrometric cells, which have a predefined geometric shape. All the micrometric meshes of the grid have the same geometric shape and define respective through holes.

By way of non-limiting example, the meshes of the grid may be substantially round, substantially square or another regular polygon shape, or substantially rectangular in shape. The preferred shape is square.

The micrometric size of the meshes is particularly advantageous as it allows the growth of the cells present in the implant site of the device and their proliferation around it.

The thickness of the polymer matrix is preferably in the range of 1 µm to 500 µm, more preferably in the range of 2 to 100 µm, and even more preferably in the range of 3 to 10 µm. Where the meshes are substantially square, the distance separating adjacent through holes is in the range of 1 µm to 100 µm, more preferably in the range of 2 to 50 µm, and even more preferably in the range of 3 to 10 µm.

In a particularly preferred embodiment, the micrometric meshes are all substantially square in shape; the longitudinal and transverse dimensions of the through hole of each mesh are both 20 µm; the thickness of the polymer matrix is 5 µm and the distance separating adjacent through holes is 3 µm.

The expression "distance separating adjacent holes" is used to indicate a distance relative to a direction perpendicular to the axis of the holes, i.e. along a plane of development of the polymeric matrix.

The surface of the polymer matrix is easily adjusted and modified during the manufacturing process to achieve the desired surface properties. The µMESH device has preferably a surface zeta potential in the range of -60 mV to +20 mV, which reflects values found in biological molecules, and which may be adapted by modifying the end groups (such as COOH or NH₂), or their ratio, on the constituent polymer(s). In this case, the end groups are conjugated, preferably by covalent binding, to specific molecules, adapted to support the binding of the µMESH device to the surrounding cells. For example, in at least one portion of a surface of the µMESH device, the end groups of the polymer(s) forming its polymer matrix are conjugated to a biomolecule chosen from nucleic acids, polypeptides, glycoproteins, carbohydrates, lipids, and combinations thereof. More specifically, by way of example, these biomolecules are chosen from natural or synthetic ligands for a cell surface receptor, such as a growth factor, hormones, LDL, transferrin, antibodies, antibody fragments, and single-chain antibodies.

In addition, the surface of the µMESH device may be modified by adding, on one side or on both sides, other integral structural parts having the object of improving the anchorage of the device, such as pillars, needle-like structures, etc.

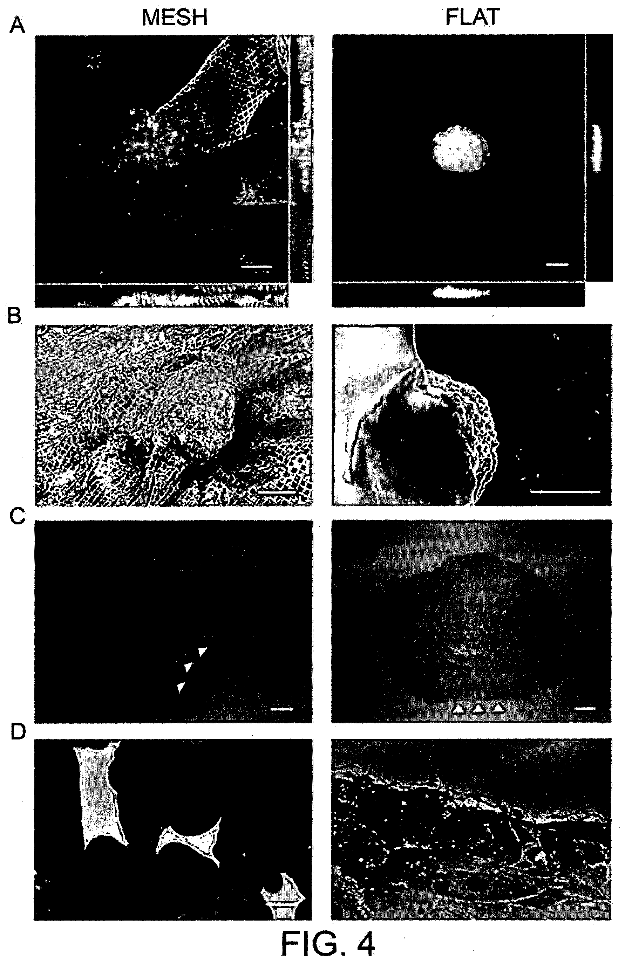

The µMESH device described above is characterized by a high degree of flexibility, allowing the device to adapt, conform, adhere and integrate to and with the surrounding tissue, without affecting the overall physico-chemical or pharmacological properties of the active ingredients incorporated therein (i.e., their incorporation and release profile). These features of the µMESH device derive from its characteristic grid shape with micrometric meshes, rather than from its surface properties. This is particularly relevant for application in the treatment of brain tumors, as inventors have shown that the close interaction between the µMESH device and the surrounding tissue promotes local recruitment of tumor cells. In particular, as will be detailed in the following examples and in particular in Figure 4, the µMESH device is advantageously able to wrap around and adapt to the tumor mass; moreover, its micrometric through holes allow the cells to migrate into and around the device. The migration of cells in and around the device may be further increased by incorporating cytokines into the µMESH device, which are able to mediate the migration of tumor cells to the same device.

Finally, the µMESH device may be easily stored in dry conditions, preserving both the polymer and the active ingredient, and preventing the latter from being released prematurely from the device. This ensures that the features and performance of µMESH devices remain homogeneous and consistent throughout the time between manufacture and use.

A second aspect of the invention is the manufacturing method of the µMESH device described above. The method consists of the following steps:
i. providing a silicon template (wafer) having the negative of the general grid shape of the µMESH device etched on a face;
ii. starting from the aforesaid silicon template, obtaining a polydimethylsiloxane (PDMS) template reproducing the general grid shape of the µMESH device;
iii. from the aforesaid polydimethylsiloxane template, obtaining a template of one or more biodegradable and biocompatible support polymers (preferably polyvinyl alcohol (PVA)) reproducing the negative of the general grid shape of the µMESH device;
iv. providing a mixture comprising one or more biodegradable and biocompatible matrix polymers and one or more pharmaceutical active ingredients;
v. pouring the mixture of step iv) on the template of step iii) and removing the template of step iii),
thus obtaining an implantable µMESH device according to the invention.

In a preferred embodiment, step i) is achieved by direct laser writing and dry etching techniques and step ii) is achieved by the replica-molding technique.

A further optional step is to conjugate, on at least one portion of a surface of the device matrix, the molecules or biomolecules mentioned above that will help bind the device to the surrounding cells when the device is implanted.

The main advantage of using the manufacturing method described above is that it is easily scalable.

A third aspect of the invention concerns the medical uses of the µMESH device. A preferred medical application is localized chemotherapy of tumors, in particular, but not limited to, brain tumors.

Brain tumors particularly suitable for treatment with the µMESH device are malignant gliomas. After surgical removal of the main tumor mass, the µMESH device is implanted at the site of the surgical resection to remove the residual tumor cells pharmacologically. The following experimental data show that the µMESH device is able to inhibit growth and J induce death of residual tumor cells of glioblastoma multiforme, thus stopping recurrences. In this application, the µMESH device of the invention is even more effective than the only similar implantable device already approved by the FDA, i.e. the Gliadel^{®}.

Without wishing to be bound to any one theory, it is believed that the µMESH device, once positioned in the target site, covers it and, due to its through holes of a size comparable with those of the cells, allows tumor cells to grow around it. Thus, the µMESH device may be fully integrated with the surrounding tissue, coming into close contact with the target cells and thus achieving greater effectiveness.

Conversely, a device without through holes (such as the implantable wafers of the prior art) may not achieve this intimate interaction with the tissues and, consequently, the growth of the tumor cells has the effect of removing or moving the device. Similarly, other devices of the prior art, such as surgical nets, which also have through holes but are larger than those of the µMESH device according to the invention, are only able to establish a superficial relationship with the cells.

The µMESH device may also be used to treat other diseases, for which a controlled localized release of the active ingredient is promising, such as atherosclerosis and diabetes.

For example, in the treatment of atherosclerosis, a µMESH device incorporating antiproliferative and anti-inflammatory agents is placed along the affected vessel through the use of a catheter or similar device, thus adhering to the vessel walls and continuously releasing antiproliferative and anti-inflammatory agents to destroy atherosclerotic plaque and prevent the formation of new lesions. In the treatment of diabetes, a µMESH device incorporating anti-diabetes agents is placed along the affected vessel through the use of a catheter or similar device, thus adhering to the vessel walls and releasing the anti-diabetes agents in a manner dependent on the concentration of glucose.

The following experimental examples are given for illustrative purposes only. Therein, reference is made to the accompanying drawings, wherein:
Figure 1 is a schematic representation of the sequential steps in the manufacturing method of an embodiment of the device of the invention;
Figures 2A and 2B show images of the device of the invention obtained by scanning electron microscopy (left) and fluorescence microscopy (right);
Figure 3 shows the comparative loading and delivery profiles of a chemotherapeutic drug obtained with the device of the invention (square points) and with a similar device (FLAT) but in the form of a flat wafer (round points);
Figure 4 shows the comparative images (in the left column the device of the invention, in the right column the FLAT device in the shape of a flat wafer) of the interaction between tissue and device, obtained by different microscopic techniques: 3D confocal microscopy (A), SEM (B), optical microscopy (C) and TEM microscopy (D);
Figure 5 shows comparative images (first row µMESH device, middle row polypropylene grid, bottom row Premilene^{®} device) of the relationship between each of these devices and a tumor spheroid, detected by fluorescence microscopy (first two columns) and SEM (last two columns);
Figure 6 shows the comparative temporal interaction of the device of the invention (top line) and the flat wafer-shaped FLAT device (bottom line) with tumor spheroids over a 24-hour period;
Figure 7 shows two comparative graphs wherein (A) shows the percentage of growth of tumor cells relative to treatment days and (B) shows the cell radius relative to treatment days;
Figure 8 shows the results of the local µMESH implantation in the treatment of an orthotopic mouse model of glioblastoma multiforme.

### EXAMPLES

### 1. Manufacturing method

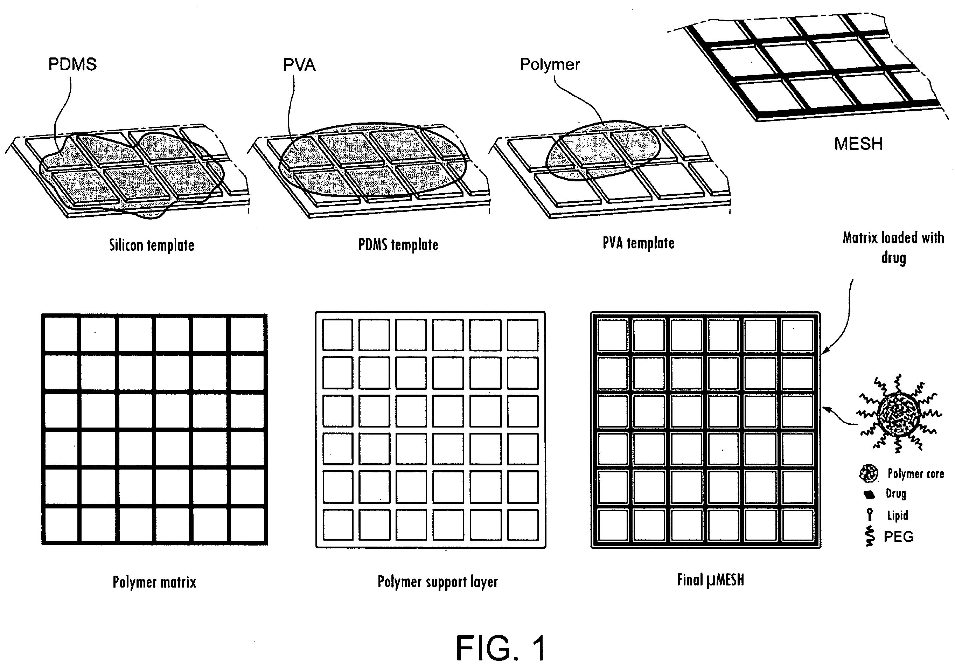

Figure 1 is a schematic representation of the steps in the manufacturing method of the µMESH device of the invention;

The µMESH device was obtained by using sequential steps for template replication. For the creation of the grid on a silicon substrate, a photopolymerizing resist was homogeneously distributed on a silicon wafer. The direct laser writing technique was used to imprint the grid pattern on the silicon. The desired thickness of the micrometric structures was then obtained by dry etching, namely Reactive Ion Etching, with consecutive steps (standard plus passivation) of the Bosch method. The silicon template was then coated with polydimethylsiloxane (PDMS), with a ratio of 1:10 between the crosslinking agent and the elastomer and polymerized in a 60°C oven for 4 hours. Subsequently, the PDMS template was replicated in polyvinyl alcohol (PVA), resulting in an identical replica of the original pattern of the silicon template, pouring a 3.5% w/v PVA solution over the PDMS and letting all the water evaporate. Then, a polymeric paste, consisting of PLGA and the active ingredient chosen to be incorporated in the device, was spread on the template, after the latter was removed from the PDMS. Finally, the µMESH device was cut into pieces of the desired size (in this case, 5x5 mm) and stored until use.

In this embodiment, the PVA replica acts as a loadable sacrificial layer, the thickness of which may be easily modulated in the manufacturing method. The thickness of the PVA layer directly affects its rate of dissolution and, therefore, the rate of release of any agent (molecules or nanoparticles) dispersed therein.

To obtain the FLAT device used in the comparative studies, an identical protocol was used, but a smooth silicon substrate was used instead of the grid pattern template used to make the µMESH device of the invention.

### 2. Microscopic characterization

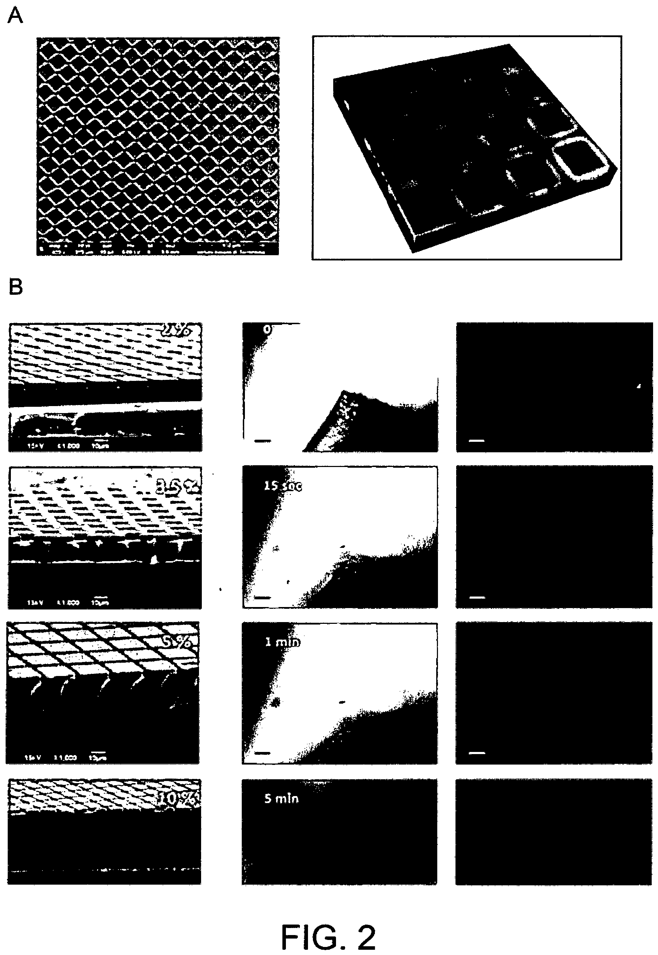

Figure 2 shows the microscopic characterization of µMESH. Representative images of the grid are shown, obtained by scanning electron microscopy (left) and fluorescence microscopy (right).

### 3. Incorporation and release profiles

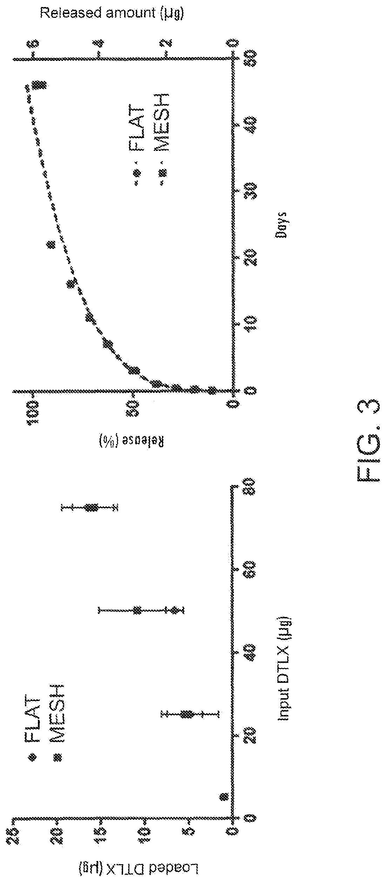

Figure 3 shows the incorporation and release profiles. In particular, a side-by-side comparison of the incorporation (left) of the active ingredient docetaxel (DTXL) and its release (right) from the µMESH device of the invention and the FLAT device for comparison is visible. The smooth FLAT device, is made of the same materials as the µMESH device (grid-shaped) and has the same overall dimensions.

Different amounts of docetaxel (DTXL), specifically 5, 25, 50 and 75 µg, were mixed into the polymeric paste and then incorporated into the µMESH, resulting in DTXL-µMESH. Three samples of DTXL-µMESH for each condition were dissolved in acetonitrile/water (1:1, v/v) and analyzed by high-performance liquid chromatography (HPLC). DTXL was eluted in water + 0.1% (v/v) trifluoroacetic acid (TFA) and acetonitrile + 0.1% (v/v) TFA, in a ratio of 47:53 v/v, under isocratic conditions, at a flow rate of 1.0 ml/min.

The good linear correlation between the amount of active ingredient mixed in the polymeric paste and the amount actually incorporated is a very important feature of the device, because it allows to finely adjust the amount of active ingredient incorporated in the device.

To study the release profile, the µMESH device and the FLAT counterpart thereof were incubated in 1 ml of phosphate buffered saline (PBS), to mimic and maximize the volume of liquid to which the devices may be exposed in a local application. At different times, the solution was centrifuged at 5000 rpm for 10 min and the supernatant was analyzed using the protocol described above. The results are expressed as a percentage of the cumulative release over time of three samples for each type of device.

### 4. Microscopic characterization of the tissue-device interaction

Empty µMESH and FLAT devices were placed on top of a PDMS coated well to maintain the correct culture conditions for tumor spheroids (i.e. in a nonadherent state). After attaching the devices, to prevent them from floating in the wells, the spheroids were placed over them and were left to grow in a suitable culture medium. In this specific case, U-87 MG cells, a representative model of glioblastoma, were chosen for spheroid formation and the devices were pre-coated with myelin (1 mg/ml in PBS) to mimic the neuronal tissue.

Figure 4 shows the integration of the tissue with the µMESH device and the FLAT wafer. In particular, this figure shows images obtained by 3D confocal microscopy (A); images obtained by SEM microscopy (B); images obtained by optical microscopy of TEM sections (C); and images obtained by TEM microscopy (D), showing the close interactions between the spheroids and the µMESH device, in contrast to the weak interaction of the FLAT wafer. The scale bar is 100 µm for all images, except for TEM images wherein the scale bar is 2 µm. The white arrows indicate the µMESH device inside the spheroid in the case of the µMESH device, or the flat side in the case of cells treated with the FLAT device.

### 5. Comparison between the µMESH device and other devices

Two grid devices were compared with the µMESH device. The first is a polypropylene grid with a rectangular structure, with a mesh thickness of approximately 250 µm, with holes approximately 400 µm wide and 800 µm high. The second is a surgical grid, Premilene^{®}, with mesh thicknesses of approximately 160 µm and non-identical holes, with dimensions of approximately 600-700 µm. These dimensions are representative of the dimensions of grid-shaped devices found on the market and are very different from the characteristic dimensions of the µMESH device. The three devices were put in contact with U87-MG tumor spheroids and their relationship therewith was evaluated over time. The ratio of Premilene^{®} to the spheroid always remains superficial and unchanged over time, unlike what happens with the µMESH device. Such behavior is similar to that of the aforementioned (FLAT device. Only the µMESH device is clearly immersed in the spheroid, as confirmed by the SEM images. The interaction of the polypropylene grid with the spheroid, on the other hand, is so weak that it cannot resist the preparation steps of the sample for electron microscopy.

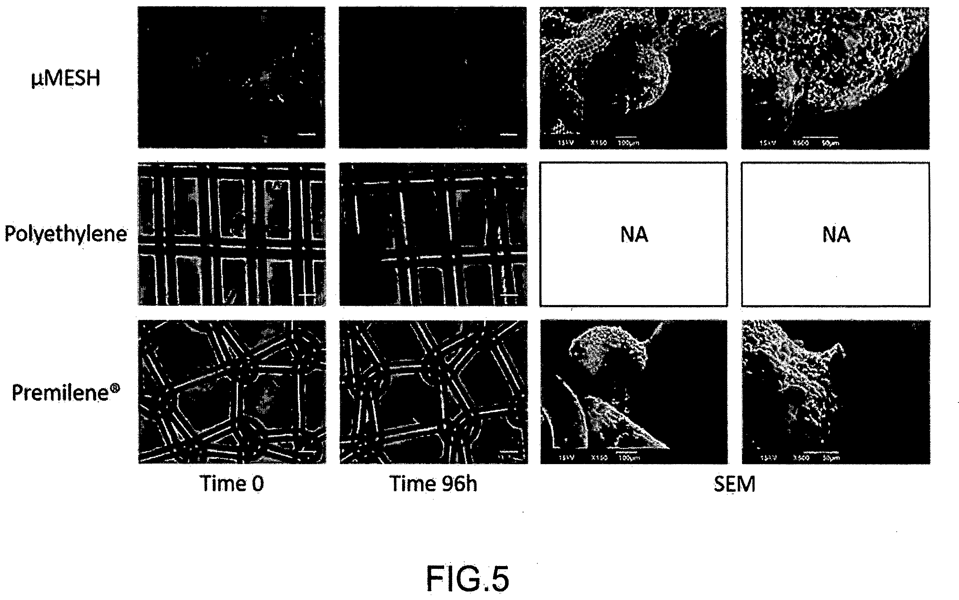

Figure 5 illustrates, by means of fluorescence microscopy (first two columns) and electron microscopy (last two columns), the interaction between a tumor spheroid and the three aforesaid devices, at the beginning and after 96 hours from the deposition of the spheroid. Fluorescence microscopy images show how, over time, only the device of the invention changes and conforms to the spheroid (the scale bar represents 250 µm fluorescence microscopy in all images). SEM images of the same devices over 96 hours show that only Premilene^{®} and µMESH establish an interaction with the spheroid, although in the first case it is only superficial, while in the second case, portions of the device of the invention that come out of the inside of the spheroid are clearly visible. This clearly demonstrates the intimate relationship established in this case between the spheroid and the µMESH device.

### 6. Anti-cancer effectiveness

DTXL-µMESH and DTXL-FLAT loaded with DTXL at a concentration of 100 nM were used to evaluate anti-tumor efficacy. The same experimental conditions used for the study of the spheroid-device interaction were used. At each observed time, the spheroids were detected and their size (in terms of area of the equatorial section and respective radius) was quantified.

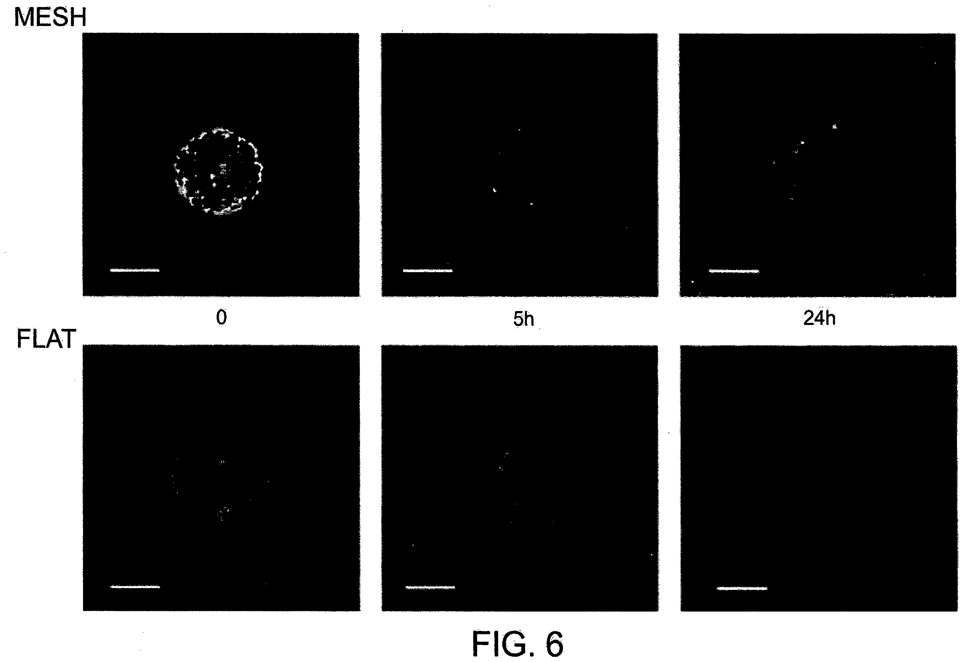

Figure 6 shows the behavior of the tumor over time with respect to its interaction with the µMESH device (upper row) or the FLAT device (lower row). The physical interaction of µMESH with tumor spheroids shows the tendency of the device to be dragged into the spheroids, providing very close interaction with the tumor, while the FLAT device does not produce this effect.

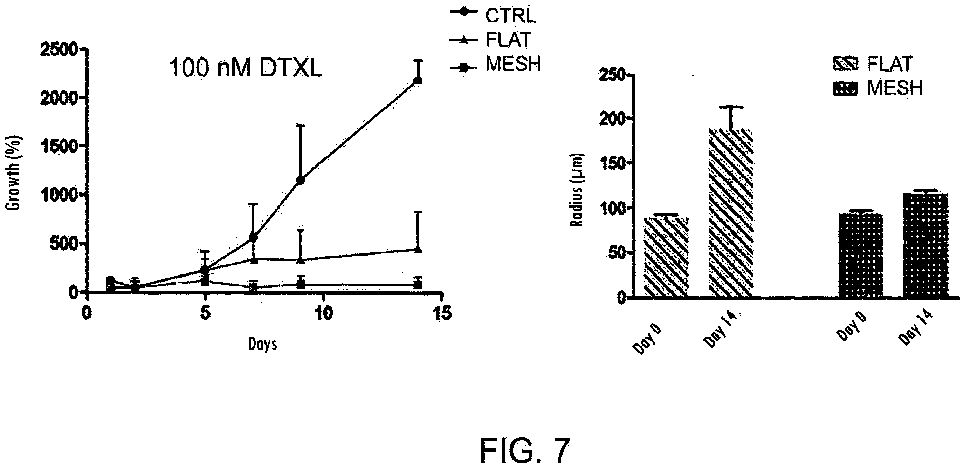

Figure 7 shows the profile of tumor growth following treatment with µMESH and FLAT devices. In particular, on the left is observable the effectiveness of DTXL-µMESH in slowing tumor growth over the period of time considered, compared with the FLAT wafer, at the same concentration and for the same time interval. On the right, the value of the mean radius on day 0 and day 14 of the spheroids treated with DTXL-µMESH or DTXL-FLAT is represented. The increased anti-tumor effectiveness of the DTXL-µMESH device is evident.

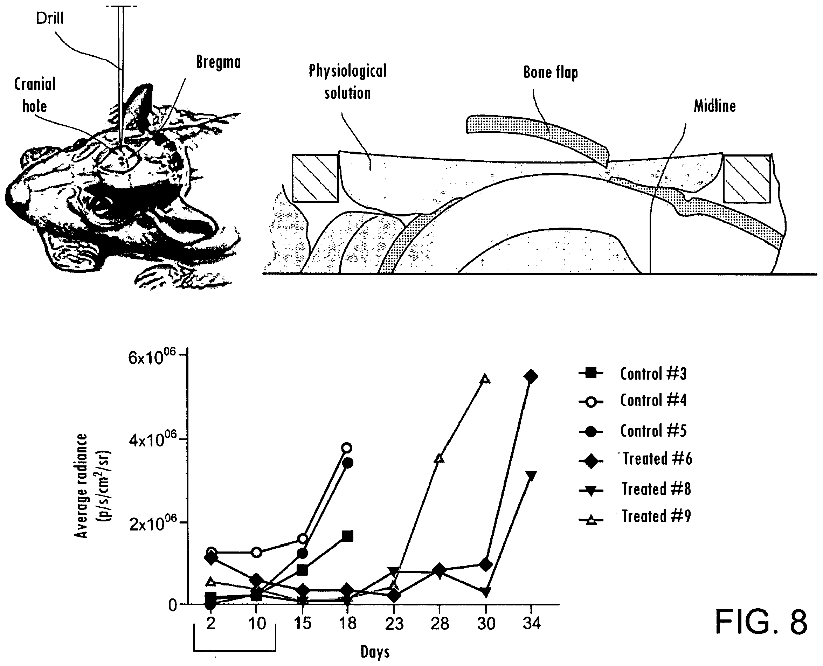

Figure 8 shows the growth profile of the tumor in the brains of mice treated with the DTXL-µMESH device. Human glioblastoma multiforme cells, luciferase-positive (U87-MG Luc⁺) mice were implanted orthotopically into the mice, as shown schematically at the top of the image. The response to treatment, which began on day 10, was measured through bioluminescence analysis, as reported in the lower image. Mice treated with DTXL-µMESH (#6, #8 and #9) show higher overall survival (about 2 times) relative to untreated mice (#3, #4 and #5).

## Claims

1. An implantable device for the localized delivery of one or more pharmaceutical active ingredients, **characterized in that** it comprises a polymeric matrix having a general grid shape comprising a plurality of micrometric meshes defining respective through holes, wherein the micrometric meshes of the grid all have the same predefined geometrical shape and wherein the size of the through holes is comprised between 1 µm and 100 µm, the polymeric matrix being made of one or more biodegradable and biocompatible polymers and including one or more pharmaceutical active ingredients.

2. The implantable device according to claim 1, wherein the micrometric meshes of the grid are essentially round, square or rectangular in shape.

3. The implantable device according to claim 1 or 2, wherein the polymeric matrix having a general grid shape has a thickness within the range of from 1 µm to 500 µm.

4. The implantable device according to any one of claims 1 to 3, wherein said one or more biodegradable and biocompatible polymers of the polymeric matrix are selected from the group consisting of poly(lactic-co-glycolic acid) (PLGA), polyethylene glycol (PEG), hyaluronic acid (HA), chitosan, polymethylmethacrylate (PMMA), polylactic acid (PLA), polyglycolic acid (PGA), polycaprolactone (PCL), and combinations thereof.

5. The implantable device according to any one of claims 1 to 4, having a surface zeta potential comprised between -60 mV and +20 mV.

6. The implantable device according to any one of claims 1 to 5, wherein at least a portion of a surface of said device has conjugated thereon molecules or biomolecules capable of favoring the binding of the device with the surrounding cells when the device is implanted.

7. The implantable device according to claim 6, wherein said molecules or biomolecules are selected from the group consisting of nucleic acids, polypeptides, glycoproteins, carbohydrates and lipids.

8. The implantable device according to any one of claims 1 to 7, wherein said one or more pharmaceutical active ingredients are anti-tumor, anti-proliferative, anti-inflammatory or anti-diabetes agents, preferably anti-tumor chemotherapeutic agents, optionally in combination with one or more further pharmaceutical active ingredients.

9. The implantable device according to any one of claims 1 to 8, wherein said one or more pharmaceutical active ingredients are included in or conjugated with nanoparticles.

10. The implantable device according to claim 9, wherein said nanoparticles are selected from the group consisting of iron oxide nanoparticles, gold nanoparticles, quantum dots, micelles, liposomes, solid lipid nanoparticles and polymeric nanoparticles.

11. The implantable device according to any one of claims 1 to 10, wherein the polymeric matrix having a general grid shape is supported on a support layer made of one or more biodegradable and biocompatible support polymers, optionally including one or more pharmaceutical active ingredients, provided that said one or more biodegradable and biocompatible polymers of the support and said one or more biodegradable and biocompatible polymers of the matrix are not the same.

12. The implantable device according to claim 11, wherein said one or more biodegradable and biocompatible polymers of the support layer are selected from the group consisting of polyvinyl alcohol (PVA), gelatin, chitosan, N-(2-Hydroxypropyl) methacrylamide (HPMA), hydroxypropyl methylcellulose (HPMC), poly(lactic-co-glycolic acid) (PLGA), polyethylene glycol (PEG), hyaluronic acid (HA), poly(methyl methacrylate) (PMMA), polylactic acid (PLA), polyglycolic acid (PGA), polycaprolactone (PCL), and combinations thereof.

13. The implantable device according to any one of claims 1 to 12, for use in the localized therapeutic treatment of a disease.

14. The implantable device according to claim 13, wherein the disease is atherosclerosis, diabetes or a tumor such as a brain tumor, wherein the brain tumor is preferably a malignant glioma.

15. A method of manufacturing an implantable device according to claim 1, comprising the steps of:
i. providing a silicon template having the negative of the general grid shape of the implantable device according to claim 1 etched on a face;
ii. starting from the silicon template of step i., obtaining a polydimethylsiloxane (PDMS) template reproducing the general grid shape of the implantable device according to claim 1;
iii. starting from the polydimethylsiloxane template of step ii., obtaining a template made of one or more biodegradable and biocompatible support polymers, reproducing the negative of the general grid shape of the implantable device according to claim 1;
iv. providing a mixture comprising one or more biodegradable and biocompatible matrix polymers and one or more pharmaceutical active ingredients;
v. pouring the mixture of step iv. on the template of step iii. and removing the template of step iii.,
thereby obtaining an implantable device according to claim 1.

16. The method of claim 15, further comprising conjugating on at least a portion of the surface of the device obtained in step v. molecules or biomolecules capable of favoring the binding of the surrounding cells when the device is implanted, the molecules or biomolecules being preferably selected from the group consisting of nucleic acids, polypeptides, glycoproteins, carbohydrates and lipids.

## Patentansprüche

1. Implantierbare Vorrichtung zur lokalen Verabreichung eines oder mehrerer pharmazeutischer Wirkstoffe, **dadurch gekennzeichnet, dass** sie eine Polymermatrix mit im Allgemeinen einer Gitterform aufweist, die eine Mehrzahl von entsprechende Durchgangslöcher ausbildenden mikrometrischen Maschen aufweist, wobei die mikrometrischen Maschen des Gitters alle dieselbe vorbestimmte geometrische Form haben und wobei die Größe der Durchgangslöcher zwischen 1 µm und 100 µm beträgt, wobei die Polymermatrix aus einem oder mehreren biologisch abbaubaren und biologisch verträglichen Polymer(en) gefertigt ist und einen oder mehrere pharmazeutische Wirkstoffe enthält.

2. Implantierbare Vorrichtung nach Anspruch 1, wobei die mikrometrischen Maschen des Gitters im Wesentlichen eine runde, quadratische oder rechteckige Form haben.

3. Implantierbare Vorrichtung nach Anspruch 1 oder 2, wobei die Polymermatrix im Allgemeinen eine Gitterform mit einer Dicke im Bereich von 1 µm bis 500 µm hat.

4. Implantierbare Vorrichtung nach einem der Ansprüche 1 bis 3, wobei das eine oder die mehreren biologisch abbaubaren und biologisch verträglichen Polymer(e) der Polymermatrix ausgewählt ist/sind aus der Gruppe bestehend aus Poly(milch-co-glykolsäure) (PLGA), Polyethylenglykol (PEG), Hyaluronsäure (HA), Chitosan, Polymethylmethacrylat (PMMA), Polymilchsäure (PLA), Polyglykolsäure (PGA), Polycaprolacton (PCL) und Kombinationen davon.

5. Implantierbare Vorrichtung nach einem der Ansprüche 1 bis 4 mit einem Oberflächen-Zeta-Potential von -60mV bis +20 mV.

6. Implantierbare Vorrichtung nach einem der Ansprüche 1 bis 5, wobei an mindestens einem Abschnitt einer Fläche der Vorrichtung Moleküle oder Biomoleküle angebunden sind, die dazu in der Lage sind, die Bindung der Vorrichtung an die umgebenden Zellen zu begünstigen, wenn die Vorrichtung implantiert ist.

7. Implantierbare Vorrichtung nach Anspruch 6, wobei die Moleküle oder Biomoleküle ausgewählt sind aus der Gruppe bestehend aus Nukleinsäuren, Polypeptiden, Glykoproteinen, Kohlenhydraten und Lipiden.

8. Implantierbare Vorrichtung nach einem der Ansprüche 1 bis 7, wobei der eine oder die mehreren pharmazeutischen Wirkstoff(e) Antitumormittel, antiproliferative Mittel, entzündungshemmende Mittel oder Antidiabetesmittel, vorzugsweise antitumorale Chemotherapeutika ist/sind, wahlweise in Kombination mit einem oder mehreren weiteren pharmazeutischen Wirkstoff(en).

9. Implantierbare Vorrichtung nach einem der Ansprüche 1 bis 8, wobei der eine oder die mehreren pharmazeutischen Wirkstoff(e) in Nanopartikeln enthalten oder mit diesen verbunden ist/sind.

10. Implantierbare Vorrichtung nach Anspruch 9, wobei die Nanopartikel ausgewählt sind aus der Gruppe bestehend aus Eisenoxid-Nanopartikeln, Gold-Nanopartikeln, Quantendots, Mizellen, Liposomen, festen Lipid-Nanopartikeln und polymeren Nanopartikeln.

11. Implantierbare Vorrichtung nach einem der Ansprüche 1 bis 10, wobei die Polymermatrix im Allgemeinen eine Gitterform hat, die an einer Stützschicht abgestützt ist, welche aus einem oder mehreren biologisch abbaubaren und biologisch verträglichen Stützpolymer(en) gefertigt ist, wahlweise einen oder mehrere pharmazeutische Wirkstoff(e) enthaltend, vorausgesetzt dass das eine oder die mehreren biologisch abbaubaren und biologisch verträglichen Polymer(e) der Stützschicht und das eine oder die mehreren biologisch abbaubaren und biologisch verträglichen Polymer(e) der Matrix nicht die gleichen sind.

12. Implantierbare Vorrichtung nach Anspruch 11, wobei das eine oder die mehreren biologisch abbaubare(n) und biologisch verträgliche(n) Polymer(e) der Stützschicht ausgewählt ist/sind aus der Gruppe bestehend aus Polyvinylalkohol (PVA), Gelatine, Chitosan, N-(2-Hydroxypropyl)methacrylamid (HPMA), Hydroxypropylmethylcellulose (HPMC), Poly(milch-co-glykolsäure) (PLGA), Polyethylengylkol (PEG), Hyaluronsäure (HA), Poly(methylmetha-crylat) (PMMA), Polymilchsäure (PLA), Polyglykolsäure (PGA), Polycaprolacton (PCL) und Kombinationen davon.

13. Implantierbare Vorrichtung nach einem der Ansprüche 1 bis 12 zur Verwendung in der lokalen therapeutischen Behandlung einer Erkrankung.

14. Implantierbare Vorrichtung nach Anspruch 13, wobei die Erkrankung Atherosklerose, Diabetes oder ein Tumor wie z.B. ein Gehirntumor ist, wobei der Gehirntumor insbesondere ein malignes Gliom ist.

15. Verfahren zum Herstellen einer implantierbaren Vorrichtung nach Anspruch 1, aufweisend die folgenden Schritte:
i. Bereitstellen einer Siliziumschablone, bei der das Negativ der gewöhnlichen Gitterform der implantierbaren Vorrichtung nach Anspruch 1 auf eine Fläche eingeprägt ist;
ii. Erhalten, ausgehend von der Siliziumschablone aus Schritt 1, einer Polydimethylsiloxan(PDMS)-Schablone, die die im Allgemeinen Gitterform der implantierbaren Vorrichtung nach Anspruch 1 nachbildet;
iii. Erhalten, ausgehend von der Polydimethylsiloxan(PDMS)-Schablone aus Schritt ii, einer Schablone, die aus einem oder mehreren biologisch abbaubaren und biologisch verträglichen Abstützpolymer(en) gefertigt ist, wodurch das Negativ der im Allgemeinen Gitterform der implantierbaren Vorrichtung nach Anspruch 1 nachgebildet wird;
iv. Bereitstellen einer Mischung, welche ein oder mehrere biologisch abbaubare und biologisch verträgliche Matrixpolymer(e) und einen oder mehrere pharmazeutische Wirkstoff(e) aufweist;
v. Ausgießen der Mischung aus Schritt iv. auf die Schablone aus Schritt iii. und Entfernen der Schablone aus Schritt iii.;
wodurch eine implantierbare Vorrichtung nach Anspruch 1 erhalten wird.

16. Verfahren nach Anspruch 15, weiter aufweisend das Verbinden von Molekülen oder Biomolekülen auf mindestens einem Abschnitt der in Schritt v. erhaltenen Fläche der Vorrichtung, wobei die Moleküle oder Biomoleküle dazu in der Lage sind, das Binden der umgebenden Zellen zu begünstigen, wenn die Vorrichtung implantiert ist, wobei die Moleküle oder Biomoleküle bevorzugt ausgewählt sind aus der Gruppe bestehend aus Nukleinsäuren, Polypeptiden, Glykoproteinen, Kohlenhydraten und Lipiden.

## Revendications

1. Dispositif implantable pour l'administration localisée d'un ou de plusieurs principes actifs pharmaceutiques, **caractérisé en ce qu'**il comprend une matrice polymère ayant une forme générale de grille comprenant une pluralité de mailles micrométriques définissant des trous traversants respectifs, dans lequel les mailles micrométriques de la grille ont toutes la même forme géométrique prédéfinie et dans lequel la taille des trous traversants est comprise entre 1 µm et 100 µm, la matrice polymère étant constituée d'un ou de plusieurs polymères biodégradables et biocompatibles et comportant un ou plusieurs principes actifs pharmaceutiques.

2. Dispositif implantable selon la revendication 1, dans lequel les mailles micrométriques de la grille sont essentiellement de forme ronde, carrée ou rectangulaire.

3. Dispositif implantable selon la revendication 1 ou 2, dans lequel la matrice polymère ayant une forme générale de grille a une épaisseur dans la plage de 1 µm à 500 µm.

4. Dispositif implantable selon l'une quelconque des revendications 1 à 3, dans lequel lesdits un ou plusieurs polymères biodégradables et biocompatibles de la matrice polymère sont sélectionnés dans le groupe constitué du poly(acide lactique-co-glycidique) (PLGA), du polyéthylène glycol (PEG), de l'acide hyaluronique (HA), du chitosane, du polyméthacrylate de méthyle (PMMA), de l'acide polylactique (PLA), de l'acide polyglycolique (PGA), de la polycaprolactone (PCL) et de combinaisons de ceux-ci.

5. Dispositif implantable selon l'une quelconque des revendications 1 à 4, ayant un potentiel zêta de surface compris entre - 60 mV et +20 mV.

6. Dispositif implantable selon l'une quelconque des revendications 1 à 5, dans lequel au moins une partie d'une surface dudit dispositif présente sur celle-ci des molécules ou biomolécules conjuguées capables de favoriser la liaison du dispositif avec les cellules environnantes lorsque le dispositif est implanté.

7. Dispositif implantable selon la revendication 6, dans lequel lesdites molécules ou biomolécules sont sélectionnées dans le groupe constitué d'acides nucléiques, de polypeptides, de glycoprotéines, de glucides et de lipides.

8. Dispositif implantable selon l'une quelconque des revendications 1 à 7, dans lequel lesdits un ou plusieurs principes actifs pharmaceutiques sont des agents antitumoraux, antiprolifératifs, antiinflammatoires ou antidiabétiques, de préférence des agents de chimiothérapie antitumoraux, éventuellement en association avec un ou plusieurs principes actifs pharmaceutiques supplémentaires.

9. Dispositif implantable selon l'une quelconque des revendications 1 à 8, dans lequel lesdits un ou plusieurs principes actifs pharmaceutiques sont inclus dans des nanoparticules ou conjugués à celles-ci.

10. Dispositif implantable selon la revendication 9, dans lequel lesdites nanoparticules sont sélectionnées dans le groupe constitué de nanoparticules d'oxyde de fer, de nanoparticules d'or, de points quantiques, de micelles, de liposomes, de nanoparticules lipidiques solides et de nanoparticules polymères.

11. Dispositif implantable selon l'une quelconque des revendications 1 à 10, dans lequel la matrice polymère ayant une forme générale de grille est supportée sur une couche de support constituée d'un ou de plusieurs polymères de support biodégradables et biocompatibles, comportant facultativement un ou plusieurs principes actifs pharmaceutiques, à condition que lesdits un ou plusieurs polymères biodégradables et biocompatibles du support et lesdits un ou plusieurs polymères biodégradables et biocompatibles de la matrice ne soient pas les mêmes.

12. Dispositif implantable selon la revendication 11, dans lequel lesdits un ou plusieurs polymères biodégradables et biocompatibles de la couche de support sont sélectionnés dans le groupe constitué de l'alcool polyvinylique (PVA), de la gélatine, du chitosane, du N-(2-hydroxypropyl) méthacrylamide (HPMA), de l'hydroxypropylméthylcellulose (HPMC), du poly(acide lactique-co-glycidique) (PLGA), du polyéthylène glycol (PEG), de l'acide hyaluronique (HA), du poly(méthacrylate de méthyle) (PMMA), de l'acide polylactique (PLA), de l'acide polyglycolique (PGA), de la polycaprolactone (PCL) et de combinaisons de ceux-ci.

13. Dispositif implantable selon l'une quelconque des revendications 1 à 12, pour une utilisation dans le traitement thérapeutique localisé d'une maladie.

14. Dispositif implantable selon la revendication 13, dans lequel la maladie est l'athérosclérose, le diabète ou une tumeur telle qu'une tumeur cérébrale, dans lequel la tumeur cérébrale est de préférence un gliome malin.

15. Procédé de fabrication d'un dispositif implantable selon la revendication 1, comprenant les étapes suivantes :
i. la fourniture d'un gabarit en silicium ayant le négatif de la forme générale de grille du dispositif implantable selon la revendication 1 gravé sur une face ;
ii. à partir du gabarit en silicium de l'étape i., l'obtention d'un gabarit en polydiméthylsiloxane (PDMS) reproduisant la forme générale de grille du dispositif implantable selon la revendication 1 ;
iii. à partir du gabarit en polydiméthylsiloxane de l'étape ii., l'obtention d'un gabarit constitué d'un ou de plusieurs polymères de support biodégradables et biocompatibles, reproduisant le négatif de la forme générale de grille du dispositif implantable selon la revendication 1 ;
iv. la fourniture d'un mélange comprenant un ou plusieurs polymères de matrice biodégradables et biocompatibles et un ou plusieurs principes actifs pharmaceutiques ;
v. le versement du mélange de l'étape iv. sur le gabarit de l'étape iii. et l'enlèvement du gabarit de l'étape iii.,
pour ainsi obtenir un dispositif implantable selon la revendication 1.

16. Procédé selon la revendication 15, comprenant en outre la conjugaison, sur au moins une partie de la surface du dispositif obtenu à l'étape v., de molécules ou biomolécules capables de favoriser la liaison du dispositif des cellules environnantes lorsque le dispositif est implanté, les molécules ou biomolécules étant de préférence sélectionnées dans le groupe constitué d'acides nucléiques, de polypeptides, de glycoprotéines, de glucides et de lipides.
